# EUROPÄISCHE PATENTSCHRIFT

(11) **EP 2 152 334 B1**
(45) Veröffentlichungstag und Bekanntmachung des Hinweises auf die Patenterteilung: **01.08.2018**
(21) Anmeldenummer: 08748355.8
(22) Anmeldetag: 16.05.2008
(51) Int. Cl.: A61M 1/00

(54) **DRAINAGEPUMPEINHEIT**
DRAINAGE PUMP UNIT
UNITÉ À POMPE DE DRAINAGE

(30) Priorität: 22.05.2007 CH 823072007
(43) Veröffentlichungstag der Anmeldung: 17.02.2010
(73) Patentinhaber: Medela Holding AG, 6340 Baar (CH)
(72) Erfinder: RAMELLA, Ivo, 6030 Ebikon (CH); JODER, Fabian, 6340 Baar (CH)
(74) Vertreter: Clerc, Natalia
(86) Internationale Anmeldenummer: PCT/CH2008/000225
(87) Internationale Veröffentlichungsnummer: WO 2008/141471

(56) Entgegenhaltungen:
- WO-A-2007/128156
- US-A- 5 466 229

## Beschreibung

### Technisches Gebiet

Die Erfindung betrifft eine Drainagepumpeinheit gemäss Oberbegriff des Patentanspruchs 1.

### Stand der Technik

Zum Absaugen von Körperflüssigkeiten bzw. -fluiden im medizinischen Bereich, beispielsweise bei bzw. nach chirurgischen Eingriffen, aber auch in der Wunddrainage, der Thoraxdrainage oder beim Absaugen von Körperfetten, werden Drainagepumpsysteme eingesetzt. Diese Drainagepumpsysteme weisen üblicherweise eine Saugpumpe, einen oder mehrere Fluidsammelbehälter und eine Drainageschlauchverbindung zwischen Patient und Fluidsammelbehälter auf. Der Fluidsammelbehälter kann am Gehäuse der Drainagepumpe lösbar befestigt sein oder mit der Pumpe über einen Vakuumschlauch verbunden sein.

Indem mittels der Saug- bzw. Vakuumpumpe im Fluidsammelbehälter ein Unterdruck erzeugt wird, wird das Fluid oder Sekret von einer Kavität des Patienten über den Drainageschlauch in den Sammelbehälter gesaugt und dort gesammelt. Am pumpenseitigen Ausgang des Sammelbehälters angeordnete Filter schützen die Saugpumpe vor einer allfälligen Verunreinigung durch das abgesaugte Fluid. Ein derartiger Fluidsammelbehälter mit starrem Deckel und daran befestigtem flexiblen Beutel ist beispielsweise aus EP 0 861 668 und WO 01/24846 bekannt. In US 5 466 229 wird eine Drainagepumpeinheit mit einem Pumpengehäuse und einem lösbar am Pumpengehäuse befestigbaren Fluidsammelbehälter offenbart.

In EP 0 466 334 wird eine Drainageleitung mit einem Drainagekatheter und einer den Katheter umgebenden luftdichten Hülle offenbart. Der Katheter ist an seinen beiden Enden mit einem Anschlussteil versehen. Am patientenseitigen Anschlussteil ist ein Anschluss für einen Gasanalysator vorgesehen.

Es ist ferner bekannt, zusätzlich zur Drainageleitung eine Serviceleitung von der Pumpe zum Patienten zu führen. In US 5 738 656 wird beispielsweise ein doppellumiger Schlauch verwendet, wobei ein Lumen die Drainageleitung bildet und das zweite Lumen eine Luftleitung ist, welche am patientenseitigen Ende in die Drainageleitung mündet. Dadurch kann in die abzusaugende Kavität des Patienten Luft oder Gas zugeführt werden und so die Kavität gespült werden. Zudem kann dieses Lumen als Messleitung benützt werden, um Strömungs- oder Druckdifferenzen festzustellen. Dadurch lässt sich der Drainagevorgang optimal überwachen und auch automatisch steuern.

Aus WO 05/061025 wird eine mit dem patientenseitigen Ende des Drainageschlauchs verbundene Serviceleitung zum Spülen der Drainageleitung verwendet, um Verschlüsse der Leitung durch angesaugte Fluidklumpen oder Gewebe zu vermeiden bzw. zu beheben.

US 6 626 827 beschreibt eine Drainageschlaucheinheit mit zwei Schläuchen, welche am pumpenseitigen Ende ein y-förmiges Anschlussteil aufweist. Am patientenseitigen Ende münden die zwei Schläuche in zwei voneinander unabhängige Anschlussteile.

US 5 029 580 offenbart eine Drainageschlaucheinheit mit einem doppellumigen Schlauch, welcher eine Drainageleitung und eine Luftzufuhrleitung beinhaltet. Am patientenseitigen Ende weist der Schlauch innere Durchgangsöffnungen auf, welche die zwei Leitungen miteinander verbinden. Dieser Schlauch ist an seinen Enden mit einem pumpenseitigen bzw. einem patientenseitigen Anschlussteil versehen. In diesen Anschlussteilen sind ferner weitere Anschlussmöglichkeiten vorgesehen.

Auch US 5 134 996 offenbart einen mehrlumigen Drainageschlauch, welcher von einer Hülle umgeben ist und welcher an seinen zwei Enden mit Anschlussteilen versehen ist. Diese Anschlüsse helfen dank ihrer Anschlussteile zwar, Fehlmanipulationen zu vermeiden. Sie sind jedoch relativ kompliziert aufgebaut, insbesondere da sie aus mehreren Einzelteilen bestehen. Zudem lassen sie sich nur mit einem doppellumigen Katheterschlauch, insbesondere nur mit einem Schlauch mit einem speziell ausgebildeten patientenseitigen Ende verwenden. Da diese Drainageschlaucheinheiten nicht mehrfach benützt werden können und als Einwegteile nach einmaligem Gebrauch weggeworfen werden, müssen sie jedoch so kostengünstig wie möglich sein.

Nachteilig bei den Systemen gemäss dem Stand der Technik ist zudem, dass die Drainageschläuche stets vom Fluidsammelbehälter entfernt werden müssen, wenn dieser geleert wird. Dies führt zu einer unnötigen Störung des Patienten.

In dem erst nach dem Prioritätstag veröffentlichten Dokument WO 2007/128156 A2 ist ein Anschlussteil offenbart, mittels welchem eine Schlaucheinheit in das Pumpengehäuse einer Absaugpumpeneinheit einsteckbar ist.

### Darstellung der Erfindung

Es ist deshalb eine Aufgabe der Erfindung, eine Drainagepumpeinheit zu schaffen, welche die Störung des Patienten auf ein Minimum reduziert.

Diese Aufgabe löst eine Drainagepumpeinheit mit den Merkmalen des Patentanspruchs 1.

Die erfindungsgemässe Drainagepumpeinheit zum Absaugen von Körperfluiden mittels einer Saugpumpe weist eine Drainagepumpvorrichtung mit einem Pumpengehäuse zur Aufnahme der Saugpumpe und einen lösbar am Pumpengehäuse befestigbaren Fluidsammelbehälter auf. Die Drainagepumpeinheit weist ferner ein pumpenseitiges Anschlussteil auf, welches einen darin verlaufenden Drainagekanal aufweist, der eine Drainagemündung zur Aufnahme des pumpenseitigen Endes eines Drainageschlauchs, insbesondere ein Verbindungselement zur Verbindung mit einem patientenseitigen Drainageschlauch, besitzt. Das Anschlussteil ist am Pumpengehäuse lösbar gehalten und es weist einen Anschlussstutzen auf, auf welchen eine Anschlussöffnung des Fluidsammelbehälters aufsteckbar ist. Alternativ dazu kann das Anschlussteil eine Anschlussöffnung aufweisen, in welche ein Anschlussstutzen des Fluidsammelbehälters einsteckbar ist. Das Verbindungselement und der Anschlussstutzen bzw. die Anschlussöffnung sind über einen im Anschlussteil verlaufenden Drainagekanal miteinander verbunden.

Da der Drainageschlauch bzw. das Anschlussteil nun nicht im Fluidsammelbehälter sondern im bzw. am Pumpengehäuse gehalten ist und mit dem Fluidsammelbehälter verbindbar ist, kann der Fluidsammelbehälter entfernt und geleert bzw. ausgewechselt werden, ohne dass der Drainageschlauch entfernt werden muss. Er kann im Pumpengehäuse stecken bleiben. Der Patient wird somit nicht belästigt, da der Drainageschlauch nicht berührt bzw. bewegt werden muss.

Die Verbindung zwischen pumpenseitigen Anschlussteil und Fluidsammelbehälter erfolgt vorzugsweise direkt, d.h. ohne Zwischenschläuche oder Zwischenleitungen. Das Pumpengehäuse weist eine Ausnehmung auf, in welche das Anschlussteil lösbar gehalten, insbesondere einsteckbar, ist. Dadurch ist das Anschlussteil sicher gehalten und wird auch beim Loslösen des Fluidsammelbehälters nicht bewegt. Die Ausnehmung befindet sich in einer Wand des Pumpengehäuses, welche dem Fluidsammelbehälter zugewandt ist. Vorzugsweise erstreckt sich die Ausnehmung bis zu einer Kante der Wand und bildet somit ein Eckstück. Vorteilhaft ist, wenn die Kante eine obere Kante ist.

Vorzugsweise ist das Anschlussteil formschlüssig in der Ausnehmung des Pumpengehäuses gehalten.

Vorzugsweise ist die Ausnehmung im Pumpengehäuse im wesentlichen quaderförmig und das Anschlussteil weist einen im wesentlichen quaderförmigen Grundkörper auf. In einer bevorzugten Ausführungsform sind das Verbindungselement und der Anschlussstutzen bzw. die Anschlussöffnung auf zwei verschiedenen, insbesondere rechtwinklig zueinander liegenden Seiten des Anschlussteils angeordnet.

Vorzugsweise ist das pumpenseitige Anschlussteil einstückig ausgebildet.

In einer bevorzugten Ausführungsform weist das pumpenseitige Anschlussteil bzw. der pumpenseitige Endstecker ein patientenseitiges Verbindungselement zur Verbindung mit einem Serviceschlauch, ein pumpenseitiges Verbindungselement zur Verbindung mit einer im Pumpengehäuse angeordneten Serviceeinheit und einen diese zwei Verbindungsmittel verbindenden, im Anschlussteil verlaufenden Servicekanal auf. Das patientenseitige Verbindungselement zur Verbindung mit dem Serviceschlauch und das Verbindungselement zur Verbindung mit dem patientenseitigen Drainageschlauch sind dabei vorzugsweise auf derselben Seite des Anschlussteils angeordnet. Auch die Verbindung zwischen pumpenseitigem Anschlussteil und Pumpengehäuse erfolgt vorzugsweise ohne Verbindungs- oder Zwischenschläuche.

Weitere vorteilhafte Ausführungsformen gehen aus den abhängigen Patentansprüchen hervor.

### Kurze Beschreibung der Zeichnungen

Im Folgenden wird der Erfindungsgegenstand anhand eines bevorzugten Ausführungsbeispiels, welches in den beiliegenden Zeichnungen dargestellt ist, erläutert. Es zeigen:
- Figur 1: eine perspektivische Darstellung einer erfindungsgemässen Drainageschlaucheinheit;
- Figur 2: eine perspektivische Darstellung durch eine in Längsrichtung geschnittene Drainageschlaucheinheit gemäss Figur 1;
- Figur 3: einen Längsschnitt durch die Drainageschlaucheinheit gemäss Figur 1;
- Figur 4: eine perspektivische Darstellung einer Drainagepumpvorrichtung mit einer Variante eines pumpenseitigen Anschlussteils der erfindungsgemässen Drainageschlaucheinheit;
- Figur 5: eine perspektivische Darstellung einer Variante des Pumpengehäuses mit seiner Aufnahme für den pumpenseitigen Anschlussteil und
- Figur 6: eine perspektivische Darstellung des Fluidsammelbehälters mit seiner Anschlussöffnung zur steckbaren Verbindung mit dem Anschlussteil.

### Wege zur Ausführung der Erfindung

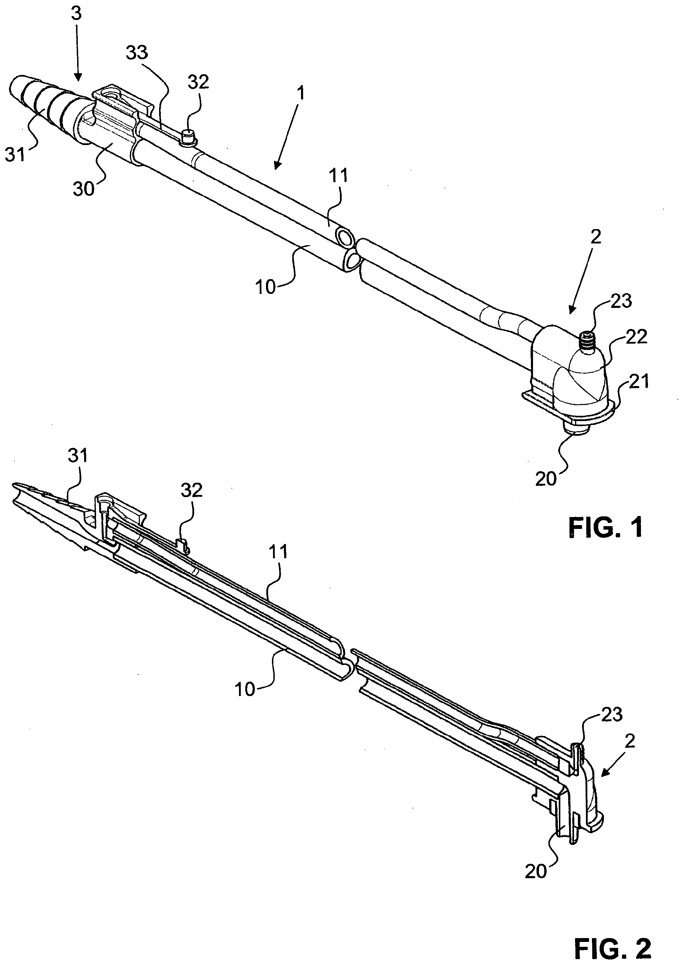

In Figur 1 ist die eine Drainageschlaucheinheit dargestellt, wie sie in den eingangs erwähnten Drainageabsaugvorrichtungen eingesetzt wird. Sie besteht im Wesentlichen aus einem Schlauchsystem 1 mit zwei oder mehreren Schläuchen 10, 11, einem pumpenseitigen Anschlussteil 2 und vorzugsweise, aber nicht zwingend, einem patientenseitigen Anschlussteil 3. Erfindungsgemäss lässt sich jedoch auch ein einzelner einlumiger Drainageschlauch einsetzen.

Die hier dargestellten Schläuche 10, 11 sind vorzugsweise einlumige, voneinander unabhängige Schläuche. Vorzugsweise sind sie aus Silikon oder PVC gefertigt. Sie verlaufen an ihren Enden getrennt voneinander. Dazwischen können sie aneinander geklebt, miteinander verschweisst oder anderweitig miteinander verbunden sein. In den Figuren sind die Schläuche nicht in ihrer gesamten Länge, sondern unterbrochen dargestellt.

Vorzugsweise weisen die zwei Schläuche unterschiedliche Durchmesser auf. Der dickere Schlauch 10 bildet dabei eine Unterdruck- und Drainageleitung zum Absaugen des Körperfluids. Der dünnere Schlauch 11 bildet eine Serviceleitung, welche beispielsweise die oben beschriebene oder eine ähnliche Druckmessung und/oder eine Reinigung der Drainageleitung ermöglicht. Beide Anwendungen sind gemeinsam aber zeitlich nacheinander möglich, wenn die Absaugeinheit am pumpenseitigen Ende der Serviceleitung beispielsweise über ein Ventil verfügt, welches für die Unterdruckmessung während des Absaugvorgangs geschlossen ist. Während des Reinigungsmodus ist das Ventil jedoch geöffnet. Die Serviceleitung kann auch auf andere bekannte Arten eingesetzt werden.

Die zwei Schläuche 10, 11 verlaufen vorzugsweise über annähernd die gesamte Länge parallel zueinander, insbesondere ihre Enden münden parallel, aber beabstandet zueinander in die jeweiligen Anschlussteile bzw. -elemente 2, 3. Unter beabstandet wird verstanden, dass sie aneinander anliegen können oder dass sie einen Freiraum zwischen sich lassen können. Sie ragen mindestens in einem der zwei Teile auf derselben Seite des Anschlussteils hinein. Die Enden sind in den Anschlussteilen 2, 3 eingesteckt, in ihnen verklebt oder anderweitig befestigt.

Im Folgenden wird zuerst das pumpenseitige Anschlussteil 2 beschrieben. Pumpenseitig meint in diesem Zusammenhang jedoch lediglich patientenfern. Das Anschlussteil kann anstatt in einem Pumpengehäuse auch in einem Fluidsammelbehälter oder einer anderen patientenfernen Einheit angeordnet sein. Wird deshalb im Folgenden von pumpenseitig gesprochen, kann ebenso behälterseitig gemeint sein.

Das pumpenseitige Anschlussteil 2 ist vorzugsweise aus Kunststoff im Spritzgussverfahren gefertigt und es ist vorzugsweise einstückig ausgebildet.

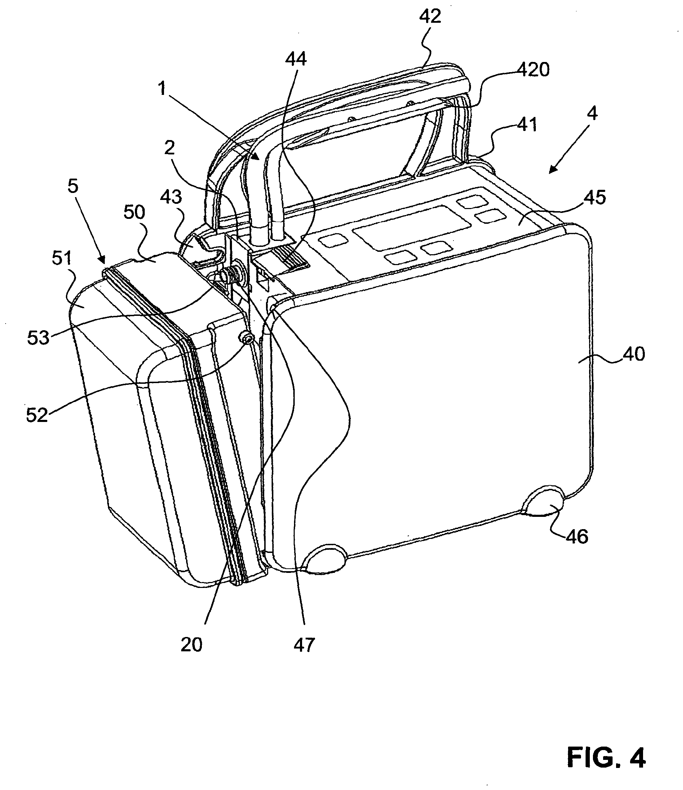

Es weist einen im Wesentlichen quaderförmigen Grundkörper 2 auf, welcher hier mit einem umlaufenden Flansch 21 versehen ist. Mit diesem Flansch 21 lässt sich das Teil 2 formschlüssig in eine entsprechende Aufnahme des Pumpengehäuses einführen und halten, wie in Figur 4 gezeigt ist.

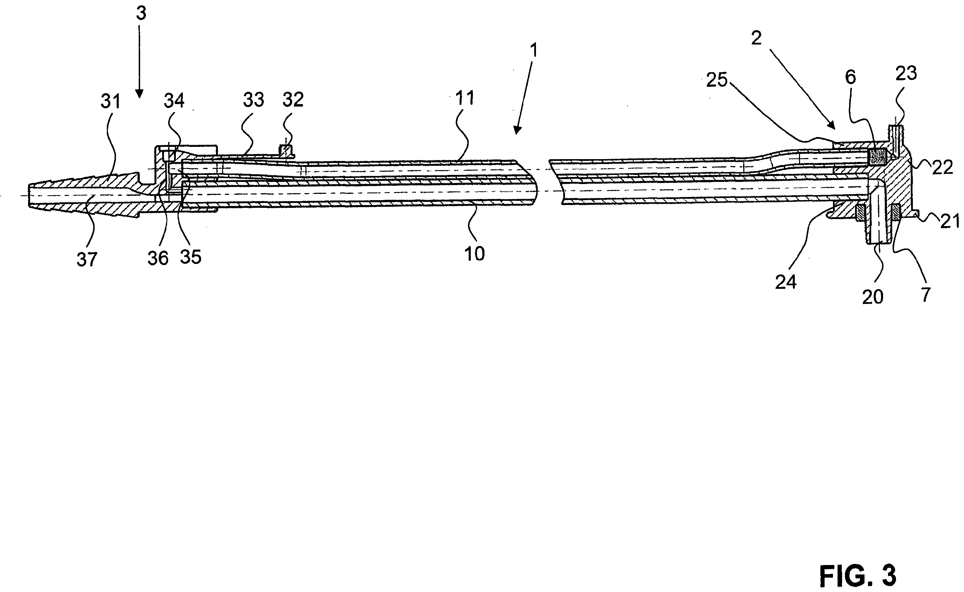

Wie in den Figuren 2 und 3 erkennbar ist, weist das Anschlussteil 2 zwei Kanäle 24, 25 auf, in deren parallel zueinander, aber voneinander beabstandet verlaufenden Mündungsöffnungen die pumpseitigen Enden des Drainage- und Serviceschlauchs 10, 11 eingesteckt sind.

Im pumpenseitigen Servicekanal 25 ist vorzugsweise ein Filter 6 angeordnet. Es handelt sich beispielsweise um ein hydrophobes und/oder um eine Bakterienfilter. Anschliessend verjüngt sich der Servicekanal 25 und biegt sich rechtwinklig zur Mündung. Er endet in einem dem Grundkörper 22 in Form eines Anschlussstutzens vorstehenden Serviceeingang 23. Dieser Serviceeingang 23 dient zur Verbindung mit einer Serviceeinheit der Saugvorrichtung.

Der pumpenseitige Drainagekanal 24 biegt sich ebenfalls rechtwinklig zu seiner Mündung und endet ebenfalls in einem dem Grundkörper 22 vorstehenden Anschlussstutzen, dem pumpenseitigen Drainageausgang 20. Dieser Ausgang 20 dient zur Verbindung mit einem Fluidsammelbehälter. Der Ausgang 20 ist hier rechtwinklig zur Mündung des Schlauchs 10 angeordnet, er kann aber auch auf einer anderen, von der Mündungsseite unterschiedlichen Seite des Grundkörpers 22 angeordnet sein. Dasselbe gilt für den Serviceeingang 23 in Bezug auf die Mündung des Serviceschlauchs 10.

Durch den Drainageausgang 20 gelangt das abgesaugte Fluid in den Behälter. Damit Dichtheit gewährleistet ist, kann im Grundkörper 22 eine umlaufende Nut um den Drainageausgang 20 vorgesehen sein. Die Nut kann mit einem Dichtring versehen sein. Vorzugsweise ist der Drainageausgang 20 auf einer Seite des Grundkörpers 22 angeordnet, welche der Seite mit dem Serviceeingang 23 gegenüberliegt.

Anstelle des hier beschriebenen pumpenseitigen Anschlussteils 2 bzw. Endsteckers lässt sich auch ein einfacher aufgebautes Teil verwenden, welches am oder im Pumpengehäuse 4 gehalten ist. Beispielsweise kann der Servicekanal und die Serviceanschlüsse entfallen, wenn nur ein einzelner Drainageschlauch und keine Serviceschläuche angeschlossen werden sollen.

Patientenseitig kann, muss aber nicht ein patientenseitiges Anschlussteil 3 vorhanden sein. Falls vorhanden, ist es vorzugsweise ebenfalls aus Kunststoff im Spritzgussverfahren hergestellt. Auch es ist vorzugsweise einstückig ausgebildet. Im folgenden wird eine Variante mit Serviceschlauch beschrieben. Es versteht sich von selber, dass das Teil einfacher, insbesondere ohne Verbindungskanal und Servicekanal ausgebildet ist, wenn kein Servicekanal, sondern nur ein einzelner Drainageschlauch angeschlossen werden soll.

Das patientenseitige Anschlussteil 3 weist einen Grundkörper 30 mit zwei parallel zueinander aber beabstandet voneinander verlaufenden Mündungen für die patientenseitigen Enden des Drainageschlauchs 10 und des Serviceschlauchs 11 auf. An diesem Grundkörper 30 ist ein patientenseitiger Drainageeingang 31 angeformt, welcher kegelförmig gestuft ausgebildet ist und sich zu seinem freien offenen Ende hin verjüngt. Er weist im Querschnitt eine Tannenbaumform auf. Der Drainageeingang 31 verläuft dabei vorzugsweise annähernd in axialer Flucht zur Mündung des patientenseitigen Endes des Drainageschlauchs 10, so dass der patientenseitige Drainagekanal 37 im Innern des Anschlussteils annähernd geradlinig verläuft.

Das patientenseitige Ende des Serviceschlauchs 11 steckt in einer Mündung eines patientenseitigen Servicekanals 35, welcher vorzugsweise einen kleineren Durchmesser aufweist als der Drainagekanal 37. Der Kanal 37 weist, wie übrigens alle anderen beschriebenen Kanäle, eine Stufe auf, welche als Anschlag für den Schlauch 11 dient. Die oben beschriebenen Mündungen werden als sich bis zu diesen Stufen erstreckend verstanden.

Der Servicekanal 23 endet im Grundkörper 30 und mündet dort in einen Verbindungskanal 36, welcher den Servicekanal 23 vorzugsweise senkrecht streift. Der Verbindungskanal 36 weist denselben oder vorzugsweise einen kleineren Durchmesser auf als der Servicekanal 35. Er endet einerseits in einem rechtwinkligen Bogen im Drainagekanal 37, vorzugsweise bei dessen Stufe zur Mündung hin. Sein anderes Ende bildet eine Öffnung 34 nach aussen, welche vorzugsweise senkrecht zu den Mündungen im Grundkörper 30 angeordnet ist.

Diese Öffnung 34 ist mit einem Verschlussdeckel 32, hier ein Stöpsel, verschlossen. In der Figur ist er noch im offenen Zustand dargestellt, wobei er vorzugsweise in dieser Konfiguration bereits geschlossen ist. Vorzugsweise wird er nämlich bereits beim Auswerfen aus der Spritzgussmaschine geschlossen, also lange bevor die Schläuche 10, 11 befestigt werden.

Vorzugsweise ist der Verschlussdeckel 32 einstückig mit dem restlichen Anschlussteil 3 hergestellt und ist deshalb, wie hier gezeigt, über ein Band 33 mit dem Grundkörper 30 verbunden. Diese Öffnung erlaubt die einstückige Herstellung dieses Anschlussteils.

In Figur 4 ist eine Drainagepumpvorrichtung dargestellt, mit welcher die Drainageschlaucheinheit vorzugsweise verwendet wird. Sie dient zum Absaugen von Körperflüssigkeiten bzw. -fluiden im medizinischen Bereich, beispielsweise bei bzw. nach chirurgischen Eingriffen, aber auch zur Wunddrainage, zur Thoraxdrainage oder zum Absaugen von Körperfetten.

Die Schlaucheinheit kann jedoch auch in anderen Drainagepumpvorrichtungen eingesetzt werden. Es ist zwar bevorzugt, aber nicht notwendig, dass dabei der Fluidsammelbehälter und im Falle von Serviceschläuchen auch die Pumpeinheit ohne weitere Zwischenleitungen mittels des pumpenseitigen Anschlussteils 2 miteinander verbunden sind.

Die hier dargestellte Drainagepumpvorrichtung weist ein Pumpengehäuse 4 auf, in welcher eine Vakuum- oder Saugpumpe und Elektronik zum Betrieb der Pumpe bzw. zum Auswerten von dank des Serviceschlauchs erhaltenen Messwerten angeordnet ist.

Das Pumpengehäuse 4 ist vorzugsweise quaderförmig ausgebildet und weist eine vordere Wand 40, eine Rückwand 41, einen Tragbügel 42 und Füsse 46 auf. Auf einer oberen Seitenwand des Gehäuses 4 ist ein Bedienfeld 45 zur Bedienung der Pumpe, vorzugsweise mit einem Display, angeordnet.

An einer seitlichen Seitenwand stehen die vordere Wand 40 und die Rückwand 41 vor und bilden eine Aufnahme für einen Fluidsammelbehälter 5. Dieser Fluidsammelbehälter 5 besteht hier vorzugsweise aus zwei Behälterhälften 50, 51 und ist aus einem durchsichtigen Kunststoff hergestellt.

Der Behälter 5 lässt sich lösbar am Pumpengehäuse 4 befestigen, vorzugsweise wird er dabei eingeschwenkt und rastet in dieser Position ein. Hierzu weisen die vordere Wand 40 und die Rückwand 41 des Pumpengehäuses 4 obere und untere Kulissenführungen auf, in welche obere und untere Befestigungsbolzen 52 des Behälters 5 eingreifen. In der Figur ist nur ein oberer Bolzen sichtbar. Die unteren Bolzen sind, wie anhand der Schrägstellung des Behälters 5 erkennbar ist, bereits eingerastet.

Der Behälter 5 weist einen Haken 53 auf, welcher zum Gehäuse 4 hin gerichtet ist und in welchen eine Kipptaste 44 des Gehäuses 4 mit einem entsprechenden Vorsprung eingreift. Dadurch ist der Behälter 5 lösbar am Gehäuse 4 fixiert.

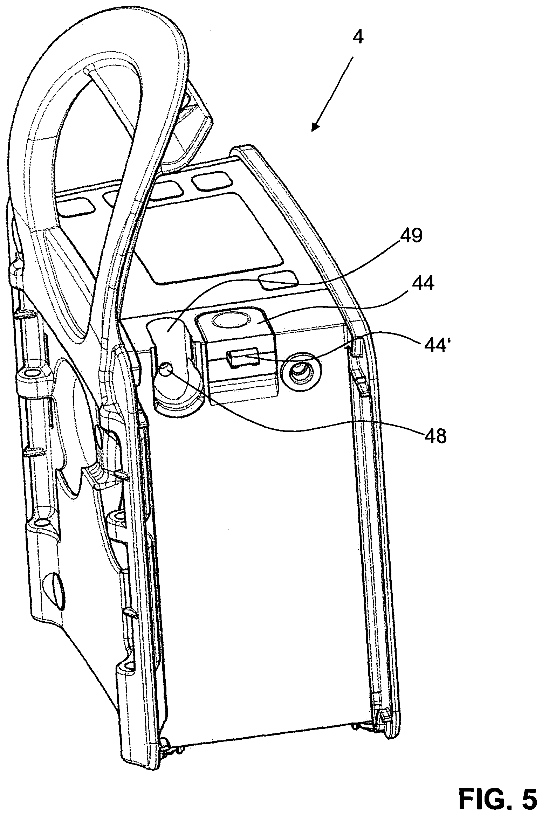

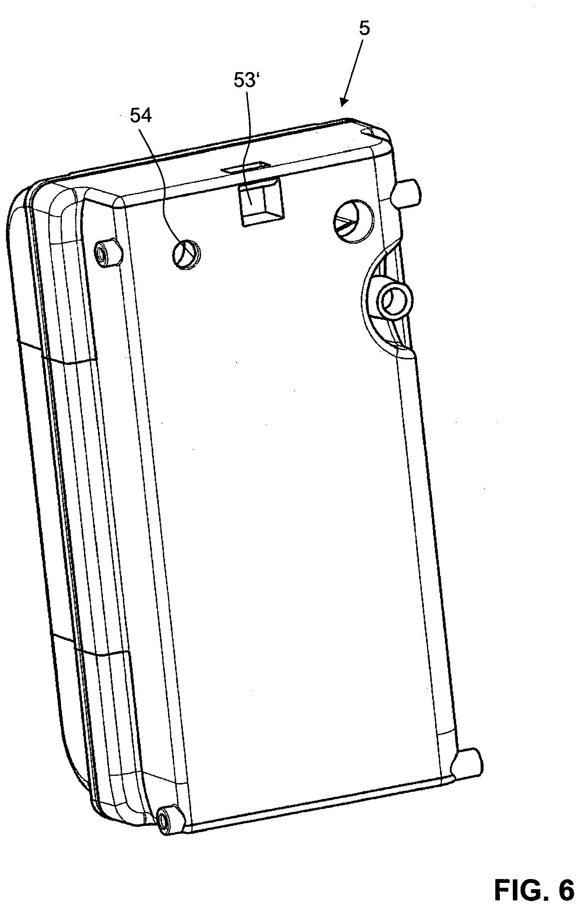

In der Variante gemäss Figur 5 weist die Kipptaste 44 einen Haken 44', welche in eine Ausnehmung 53' des Fluidsammelbehälters 5 gemäss Figur 6 eingreift.

Am Gehäuse 4 ist, dem Behälter 5 zugewandt, ein Sauganschluss 47 vorhanden. Er weist die Form eines Stutzens auf, welcher in eine entsprechende Öffnung des Behälters 5 eingreift. Dadurch lässt sich im Behälter 5 mittels der Saugpumpe ein Unterdruck erzeugen.

Im Gehäuse 4 ist ferner eine im wesentlichen quaderförmige Ausnehmung 49 vorhanden, in welche das pumpenseitige Anschlussteil 2 der Drainageschlaucheinheit einsteckbar ist und in diesem formschlüssig lösbar gehalten ist. In der in Figur 4 dargestellten Variante weist die Ausnehmung 48 plane Seitenwände auf, so dass auch das pumpenseitige Anschlussteil im Gegensatz zu dem in den Figuren 1 bis 3 dargestellten Anschlussteil plane Wände aufweisen sollte.

Die Variante gemäss Figur 5 weist die Ausnehmung 49 zwei parallel zueinander verlaufende plane und eine diese verbindende gebogene, konkave Seitenwand auf. Diese Ausnehmung 49 passt zur Ausführungsform gemäss den Figuren 1 bis 3.

Der behälterseitige Drainageausgang 21 des Anschlussteils 2 ist in beiden Varianten zum Behälter 5 hingerichtet. Durch ihn gelangt das abgesaugte Fluid in den Behälter 5. Wie in Figur 6 erkennbar ist, weist der Fluidsammelbehälter 5 eine entsprechende Anschlussöffnung 54 auf. Alternativ kann auch das Anschlussteil 2 mit einer Öffnung und der Fluidsammelbehälter mit einem dazu passenden Stutzen versehen sein. Auf jeden Fall wird eine dichtende Verbindung erstellt, wobei auf mindestens einer Seite, d.h. auf der Seite des Anschlussteils oder auf der Seite des Fluidsammebehälters, vorzugsweise ein Dichtmittel vorgesehen ist. Vorzugsweise ist das Dichtmittel ein Dichtring aus einem elastomeren Material. Hier ist es ein elastomerer Dichtring 7, wie er in Figur 3 sichtbar ist. Andere bekannte Arten, um eine Steckverbindung luft- und flüssigkeitsdicht zu gestalten, sind jedoch möglich.

Rechtwinklig dazu mündet das Schlauchsystem 1 mit den zwei Schläuchen 10, 11 in das pumpenseitige Anschlussteil 2. Das Schlauchsystem 1 ist in diesem Beispiel entlang des Gehäuses 4 geführt in einer am Tragbügel 42 angeordneten Rinne 420 gehalten.

In Figur 5 ist erkennbar, dass das Pumpengehäuse 4 eine Serviceöffnung 48 aufweist zur Aufnahme des Serviceeingangs 23 des pumpenseitigen Anschlussteils 2. Der Serviceeingang 23 des Anschlussteils 2 ragt in das Pumpengehäuse 4 hinein und ist mit einer entsprechenden Steuerungs- und/oder Auswerteinheit verbunden. Je nach Anwendung ist auch hier eine luft- bzw. flüssigkeitsdichte Verbindung vorhanden. Auch hier können Stutzen und Öffnung wiederum vertauscht sein.

Varianten zum oben genannten Beispiel sind denkbar. So kann das pumpenseitige Anschlussteil beispielsweise an einer anderen Stelle im Gehäuse eingesteckt oder an diesem gehalten sein. Die Anschlüsse können beispielsweise in einer anderen Winkellage zueinander liegen. Das pumpenseitige Anschlussteil kann beispielsweise konisch, zylindrisch oder anderweitig geeignet geformt ausgebildet sein.

Die erfindungsgemässe Drainagepumpeinheit ermöglicht ein Austauschen bzw. Leeren des Fluidsammelbehälters ohne Entfernung des Drainageschlauchs und somit ohne Störung des Patienten.

### Bezugszeichenliste

- 1: Schlauchsystem
- 10: Drainageschlauch
- 11: Serviceschlauch

- 2: pumpenseitiges Anschlussteil
- 20: pumpenseitiger Drainageausgang
- 21: Flansch
- 22: Grundkörper
- 23: pumpenseitiger Serviceeingang
- 24: pumpenseitiger Drainagekanal
- 25: pumpenseitiger Servicekanal

- 3: patientenseitiges Anschlussteil
- 30: Grundkörper
- 31: patientenseitiger Drainageeingang
- 32: Verschlussdeckel
- 33: Band
- 34: Öffnung
- 35: patientenseitiger Servicekanal
- 36: Verbindungskanal
- 37: patientenseitiger Drainagekanal

- 4: Pumpengehäuse
- 40: vordere Wand
- 41: Rückwand
- 42: Tragbügel
- 420: Rinne
- 43: Kulissenführung
- 44: Kipptaste
- 44': Haken
- 45: Bedienfeld
- 46: Fuss
- 47: Sauganschluss
- 48: Serviceeingang
- 49: Ausnehmung

- 5: Fluidsammelbehälter
- 50: erste Behälterhälfte
- 51: zweite Behälterhälfte
- 52: Befestigungsbolzen
- 53: Haken
- 53': Ausnehmung
- 54: Anschlussöffnung

- 6: Filter

- 7: Dichtring

## Patentansprüche

1. Drainagepumpeinheit zum Absaugen von Körperfluiden mittels einer Saugpumpe, wobei die Drainagepumpeinheit eine Drainagepumpvorrichtung mit einem Pumpengehäuse (4) zur Aufnahme der Saugpumpe und einen lösbar am Pumpengehäuse (4) befestigbaren Fluidsammelbehälter (5) aufweist, **dadurch gekennzeichnet, dass** das Pumpengehäuse (4) eine Ausnehmung aufweist, die sich in einer Wand des Pumpengehäuses (4) befindet, welche dem Fluidsammelbehälter (5) zugewandt ist, und dass die Drainagepumpeinheit ferner ein pumpenseitiges Anschlussteil (2) aufweist, wobei das Anschlussteil (2) in der Ausnehmung des Pumpengehäuses (4) lösbar gehalten ist, wobei ein im Anschlussteil (2) verlaufender Drainagekanal (24) vorhanden ist, der eine Drainagemündung zur Aufnahme des pumpenseitigen Endes eines Drainageschlauchs (10) und einen Ausgang (20) aufweist, welcher zur Verbindung mit dem Fluidsammelbehälter (5) dient.

2. Drainagepumpeinheit gemäss Anspruch 1, wobei das Anschlussteil (2) in die Ausnehmung einsteckbar ist.

3. Drainagepumpeinheit nach Anspruch 2, wobei sich die Ausnehmung bis zu einer Kante der Wand erstreckt und somit ein Eckstück bildet, wobei die Kante vorzugsweise eine obere Kante ist.

4. Drainagepumpeinheit nach einem der Ansprüche 1 bis 3, wobei das Anschlussteil (2) formschlüssig in der Ausnehmung des Pumpengehäuses (4) gehalten ist.

5. Drainagepumpeinheit nach einem der Ansprüche 1 bis 4, wobei die Ausnehmung im Pumpengehäuse (4) im wesentlichen quaderförmig ist und das Anschlussteil (2) einen im wesentlichen quaderförmigen Grundkörper aufweist.

6. Drainagepumpeinheit nach einem der Ansprüche 1 bis 5, wobei die Drainagemündung und der Ausgang (20) auf zwei verschiedenen, insbesondere rechtwinklig zueinander liegenden Seiten des Anschlussteils (2) angeordnet sind.

7. Drainagepumpeinheit nach einem der Ansprüche 1 bis 6, wobei das Anschlussteil (2) einstückig ausgebildet ist.

8. Drainagepumpeinheit nach einem der Ansprüche 1 bis 7, wobei ausserdem ein im Anschlussteil (2) verlaufender Servicekanal (35) vorhanden ist, der eine Servicemündung zur Aufnahme des pumpenseitigen Endes eines Serviceschlauchs (11) und einen Serviceeingang (23) zur Verbindung mit einer im Pumpengehäuse (4) angeordneten Serviceeinheit aufweist, wobei der Serviceeingang (23) bevorzugt an einem anderen Ende als die Servicemündung angeordnet ist.

9. Drainagepumpeinheit nach Anspruch 8, wobei das Anschlussteil (2) ein patientenseitiges Verbindungselement zur Verbindung mit dem Serviceschlauch (11), ein pumpenseitiges Verbindungselement (23) zur Verbindung mit der im Pumpengehäuse (4) angeordneten Serviceeinheit aufweist, wobei der im Anschlussteil (2) verlaufende Servicekanal (25) diese zwei Verbindungsmittel miteinander verbindet.

10. Drainagepumpeinheit nach Anspruch 9, wobei das patientenseitige Verbindungselement zur Verbindung mit dem Serviceschlauch (11) und die Drainagemündung zur Aufnahme des patientenseitigen Drainageschlauchs (10) auf derselben Seite des Anschlussteils (2) angeordnet sind.

## Claims

1. Drainage pump unit for aspirating body fluids by means of a suction pump, the drainage pump unit comprising a drainage pump device with a pump housing (4) for receiving the suction pump, and a fluid collection container (5) that can be secured releasably on the pump housing (4), **characterized in that** the pump housing (4) has a recess being located in a wall of the pump housing (4) directed towards the fluid collection container (5), and **in that** the drainage pump unit also comprises a pump-side attachment part (2), wherein the attachment part (2) is held releasably in the recess of the pump housing (4), in which a drainage channel (24) extending through the attachment part (2) is provided which has a drainage mouth for receipt of the pump-side end of a drainage tube (10) and an outlet (20) which serves for connection to the fluid collection container (5).

2. Drainage pump unit according to Claim 1, in which the attachment part (2) can be plugged into the recess.

3. Drainage pump unit according to Claim 2, in which the recess extends as far as an edge of the wall and thus forms a corner piece, the edge preferably being an upper edge.

4. Drainage pump unit according to one of Claims 1 to 3, in which the attachment part (2) is hold in a form-fit engagement in the recess of the pump housing (4).

5. Drainage pump unit according to one of Claims 1 to 4, in which the recess in the pump housing (4) has a substantially cuboid shape, and the attachment part (2) has a substantially cuboid main body.

6. Drainage pump unit according to one of Claims 1 to 5, in which the drainage mouth and the outlet (20) are arranged on two different sides of the attachment part (2), in particular on two sides lying at right angles to each other.

7. Drainage pump unit according to one of Claims 1 to 6, in which the attachment part (2) is designed in one piece.

8. Drainage pump unit according to one of Claims 1 to 7, in which a service channel (25) extending through the attachment part (2) is further provided which has a service mouth for receipt of the pump-side end of a service tube (11) and a service inlet (23) for connection to a service unit arranged in the pump housing (4), in which the service inlet (23) is preferably arranged on another end than the service mouth.

9. Drainage pump unit according to Claim 8, in which the attachment part (2) has a patient-side connection element for connection to the service tube (11), a pump-side connection element (23) for connection to the service unit arranged in the pump housing (4), in which service channel (25) extending through the attachment part (2) connects these two connection means with each other.

10. Drainage pump unit according to Claim 9, in which the patient-side connection element for connection to the service tube (11) and the drainage mouth for receipt of the patient-side drainage tube (10) are arranged on the same side of the attachment part (2).

## Revendications

1. Unité à pompe de drainage destinée à l'aspiration de fluides corporels au moyen d'une pompe d'aspiration, où l'unité à pompe de drainage présente un dispositif à pompe de drainage ayant un boitier de pompe (4) pour recevoir la pompe d'aspiration et un récipient de collecte de fluides (5) pouvant être fixé au boitier de pompe (4), **caractérisé en ce que** le boitier de pompe (4) présente un évidement, se trouvant dans une paroi du boitier de pompe (4), faisant face au récipient de collecte de fluides (5), et **en ce que** l'l'unité à pompe de drainage présente en outre une pièce de raccord côté pompe (2), où la pièce de raccord (2) est maintenue de manière libérable dans l'évidement du boitier de pompe (4), où un canal de drainage (24) s'étendant dans la pièce de raccord (2) est prévu, qui présente une embouchure de drainage pour recevoir l'extrémité côté pompe du tuyau de drainage (10) et une sortie, qui sert à la liaison avec le récipient de de collecte de fluides (5).

2. Unité de pompe de drainage selon la revendication 1, où la pièce de raccord (2) peut être emboitée dans l'évidement.

3. Unité à pompe de drainage selon la revendication 2, où l'évidement s'étend jusqu'à un bord de la paroi et forme ainsi une pièce d'angle, où le bord est préférablement un bord supérieur.

4. Unité à pompe de drainage selon une des revendications 1 à 3, où la pièce de raccord (2) est maintenue dans l'évidement du boîtier de pompe (4) de manière complémentaire en forme.

5. Unité à pompe de drainage selon une des revendications 1 à 4, où l'évidement dans le boitier de pompe (4) est essentiellement en forme de parallélépipède et la pièce de raccord (2) présente un corps de base sensiblement en forme de parallélépipède.

6. Unité à pompe de drainage selon une des revendications 1 à 5, où l'embouchure de drainage et la sortie (20) sont disposées sur deux côtés différents, en particulier perpendiculaires l'un par rapport à l'autre, de la pièce de raccord (2).

7. Unité de pompe de drainage selon une des revendications 1 à 6, où la partie de raccord (2) est formée en une seule pièce.

8. Unité à pompe de drainage selon une des revendications 1 à 7, où en outre un canal d'entretien (35) s'étendant dans la pièce de raccord (2) est prévu, qui présente une ouverture d'entretien pour recevoir l'extrémité côté pompe d'un tuyau d'entretien (11) et une entrée d'entretien (23) pour une liaison avec une unité d'entretien disposée dans le boîtier de pompe (4), où l'entrée d'entretien (23) est préférablement disposée sur une autre extrémité que l'ouverture d'entretien.

9. Unité à pompe de drainage selon la revendication 8, où la pièce de raccord (2) présent un élément de liaison côté patient pour une liaison avec le tuyau d'entretien (11), un élément de liaison côté pompe (23) pour une liaison avec l'unité d'entretien disposée dans le boitier de pompe (4), où le canal d'entretien (25) s'étendant dans la pièce de raccord (2) relie ces deux moyens de liaison l'un avec l'autre.

10. Unité à pompe de drainage selon la revendication 9, où l'élément de liaison côté patient pour une liaison avec le tuyau d'entretien (11) et l'embouchure de drainage pour recevoir le tuyau de drainage côté patient (10) sont disposés sur le même côté de la pièce de raccord (2).
